# EUROPEAN PATENT SPECIFICATION

(11) **EP 4 041 194 B1**
(45) Date of publication and mention of the grant of the patent: **13.03.2024**
(21) Application number: 21718127.0
(22) Date of filing: 15.04.2021
(51) Int. Cl.: A61K 9/00, A61K 9/08, A61K 47/18, A61P 35/00, A61K 9/19, A61K 31/4995

(54) **COMPOSITION COMPRISING TRABECTEDIN AND AN AMINO ACID**
ZUSAMMENSETZUNG MIT TRABECTEDIN UND EINER AMINOSÄURE
COMPOSITION COMPRENANT DE LA TRABECTÉDINE ET UN ACIDE AMINÉ

(30) Priority: 15.04.2020 EP 20169567
(43) Date of publication of application: 17.08.2022
(73) Proprietor: EVER Valinject GmbH, 4866 Unterach am Attersee (AT)
(72) Inventor: ACHLEITNER, Maria-Lena, 4866 Unterach am Attersee (AT); GEHWOLF, Nikolaus, 4866 Unterach am Attersee (AT); SCHNAIT, Heinz, 4863 Seewalchen am Attersee (AT)
(74) Representative: Gassner, Birgitta
(86) International application number: PCT/EP2021/059772
(87) International publication number: WO 2021/209545

## Description

### Field of the Invention

The present invention relates to a composition, a lyophilized formulation and an intravenous injection comprising trabectedin and an amino acid.

### Background Art

Trabectedin (Ecteinascidin or ET-743) is a tetrahydroisoquinoline alkaloid, which was initially isolated from the marine tunicate *Ecteinascidia turbinata* and possesses antitumor activity.

Trabectedin has limited aqueous solubility and thermal stability, representing a challenge for the development of formulations for medical purposes. Trabectedin is particularly effective in the treatment of sarcoma and ovarian cancer.

WO2000069441 discloses the use of trabectedin in the preparation of a pharmaceutical composition for the treatment of cancer in humans. The pharmaceutical composition is formulated as a lyophilized product containing mannitol as a bulking agent and a phosphate buffer at pH 4 in order to stabilize trabectedin. The formulation is administered as intravenous infusion.

WO2006046079 discloses that disaccharides stabilize ecteinascidin formulations. WO2006046079 describes a composition comprising an ecteinascidin such as ET-743 and a disaccharide such as sucrose, which is lyophilized and reconstituted to obtain an intravenous infusion.

Trabectedin is currently formulated as a sterile lyophilized product provided in a vial containing 0.25 mg trabectedin, 100 mg sucrose and 2 mg potassium in the form of potassium dihydrogen phosphate, as well as potassium hydroxide and phosphoric acid for pH adjustment.

WO2017133544A1 refers to stabilized trabectedin formulations which may comprise glucose as a first excipient, and hydroxyethyl starch, dextran, or sodium carboxymethyl cellulose or hydroxypropyl beta cyclodextrin as second excipient.

IN201741041173 discloses a stable pharmaceutical composition of trabectedin. Exemplified is a formulation comprising trabectedin and L-arginine in a ratio of 1:400. Such a high amount of amino acid leads to high solution volume for lyophilization and consecutively to longer drying cycles. Additionally, high amounts of auxiliary additives may lead to negative impacts to the patients.

In view of the importance of trabectedin as a chemotherapeutic agent, there is still a need for improved formulations of trabectedin, which are biocompatible, stable and suitable for medical purposes.

### Summary of Invention

It is the objective of the present invention to provide a formulation of trabectedin which is stable and suitable for medical purposes.

The objective is solved by the claimed subject matter and as disclosed herein.

The present invention provides a composition comprising trabectedin and at least one amino acid, wherein the weight ratio (w/w) of trabectedin to amino acid is 1:50 to 1:100, and wherein the amino acid is L-arginine.

In one embodiment the weight ratio (w/w) of trabectedin to amino acid is about 1:70, and wherein the amino acid is L-arginine.

In one aspect, the composition described herein comprises a buffering agent. The buffering agent may be selected from the group consisting of citric acid, phosphoric acid, acetic acid, basic amino acid and sodium hydroxide, or any mixture thereof, or a mixture of citric acid, phosphoric acid and optionally a basic amino acid.

In one aspect, the composition described herein comprises further substances selected from the group comprising a complexing agent such as ethylenediaminetetraacetic acid (EDTA); an antioxidant such as monothioglycerol; a surface-active agent such as polysorbate; mannitol; and ascorbic acid.

In the formulation according to the invention, the primary source of pH control is a buffering agent. Typically, the buffering agent is present as an acid or a base and its conjugate base or acid, respectively. In one embodiment, the range of buffering salt is 1-100 mM, preferably between 5-50 mM, most preferentially about 10 mM (in solid formulations, the amount of buffer is selected to produce this concentration after reconstitution/dilution). The concentration of buffer and the pH of the solution are advantageously chosen to give optimal balance of solubility and stability. Examples of suitable buffers include mixtures of weak acids and alkali metal salts (e.g., sodium, potassium) of the conjugate base of weak acids such as sodium citrate and disodium hydrogen phosphate.

In one embodiment, the composition is in the form of a lyophilized formulation. The present invention therefore also provides a lyophilized formulation comprising trabectedin, at least one amino acid, wherein the weight ratio (w/w) of trabectedin to amino acid is 1:50 to 1:100, and wherein said amino acid is L-arginine. In a specific embodiment the weight ratio (w/w) of trabectedin to amino acid is about 1:70, and wherein the amino acid is L-arginine.

In a further embodiment the lyophilized formulation comprises a buffering agent.

In one aspect, the amino acid in the lyophilized formulation is L-arginine and the buffering agent is citric acid or phosphoric acid, or a mixture thereof.

In another aspect, the lyophilized formulation is provided in a vial. In an exemplary embodiment, the vial contains 0.1 to 1 mg trabectedin, 10 to 60 mg L-arginine, 0.5 to 8 mg citric acid, 5 to 40 mg phosphoric acid. In a specific embodiment, the vial contains 0.25 mg trabectedin, 17.4 mg L-arginine, 1.9 mg citric acid, and 10 to 15 mg phosphoric acid.

The lyophilized formulation is suitable for preparing an intravenous infusion solution by reconstitution in an aqueous medium. Therefore, the invention further provides an intravenous infusion solution comprising trabectedin, an amino acid, a buffering agent and water for injection.

In one aspect, the intravenous infusion solution is used in the treatment of cancer. The cancer to be treated may be sarcoma, selected from the group of leiomyosarcoma, liposarcoma, osteosarcoma, ovarian cancer, breast cancer, melanoma, colorectal cancer, mesothelioma, renal cancer, endometrial cancer and lung cancer, or any combination thereof.

In a further embodiment, the intravenous infusion described herein is used for the treatment of adult patients with advanced soft tissue sarcoma.

In one embodiment, the intravenous infusion solution is used for the treatment of patients with relapsed platinum-sensitive ovarian cancer. The treatment may be in combination with pegylated liposomal doxorubicin (PLD).

### Brief Description of Drawings

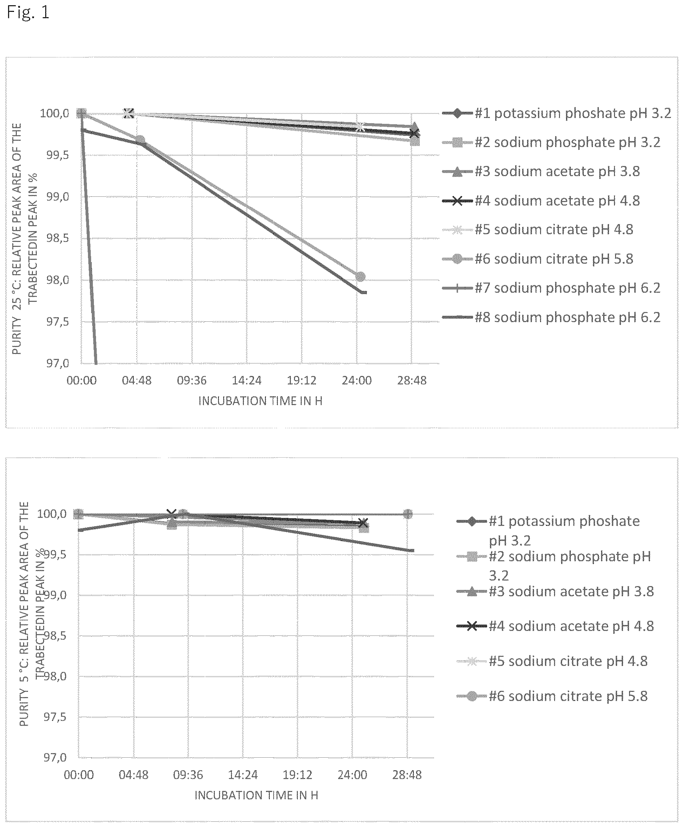

Fig. 1: Purity of the short time stability samples stored at 5 ° C (top) and 22 ° C (bottom) for up to 29 h.
Fig. 2: Stability of lyophilizates at 2-8 ° C: content of trabectedin (A285 nm) by RP-HPLC in % of the reference (0.25 mg/mL).
Fig. 3: Stability of lyophilizates at 2-8 ° C: purity of the samples as relative peak area of the trabectedin peak by RP-HPLC analysis (A285 nm).

### Description of Embodiments

The present invention relates to a composition comprising trabectedin and at least one amino acid. The formulation comprises trabectedin as active substance and may be formulated as a lyophilized formulation, which may be reconstituted to obtain an intravenous infusion.

In the context of the present invention, trabectedin can be of natural, semisynthetic or synthetic origin, including combinations of origins.

Known formulations of trabectedin use saccharides (e.g., WO2017133544 A1) to obtain a stable formulation. However, saccharides may show several unfavorable properties when used in pharmaceutical formulations. First of all, glucose and saccharose cannot be administered to diabetic patients without taking the additional carbohydrate intake into consideration. Diabetic patients are required to control their intake of glucose-generating carbohydrates and their glucose-lowering medication to keep their blood glucose level within certain boundaries. Therefore, a drug formulation containing glucose or saccharose is not optimal for this specific patient group.

Furthermore, saccharides are also the source for a specific degradation product of trabectedin that will be formed. All saccharides that can form a hemiacetal structure in solution (i.e., all "reducing" sugars) can react with hydroxy groups to from a stable acetal and water (Jerry March, Advanced Organic Chemistry, 3rd ed. Pages 789 - 790; John Wiley & Sons). The reaction products, acetal and water are in equilibrium with the drug compound, e.g., trabectedin and its hydroxy group. Under removal of water (e.g., after freeze-drying) the equilibrium is shifted far to the acetal side thus forming this reaction product in relevant quantities.

Trabectedin, whose structure shows such a hydroxy group, is therefore prone to form acetals with reducing sugars, like glucose or lactose, during freeze-drying.

This specific degradation pathway has been observed on freeze-dried formulations of trabectedin containing glucose. When such formulations are freeze-dried, the formation of the trabectedin-glucose acetal was observed. Although, this acetal hydrolyzes on reconstitution of the lyophilizate, it is still present in reconstituted solution for hours (e.g., with relative levels of about 0.5 % after 6 hours). It can be detected by HPLC. The HPLC method employed is identical with the one described herein in the formulation screening section. The degradation finally leads to an equilibrium with quite low, but still detectable levels of the acetal impurity. Applying a drug product which contains a degradation product of unknown toxicological properties to patients may be problematic from a toxicological point of view but also from a dose accuracy point of view because part of the trabectedin dose is lost in the acetal and not available for therapy.

This side effect of trabectedin formulations containing reducing sugars (e.g., glucose or lactose) can be overcome by simply waiting after preparation of an infusion solution until most of the acetal has been hydrolyzed before infusion is started. However, in hospital practice this is a time consuming and less desirable procedure that should be avoided. Therefore, a formulation that does not show this kind of degradation product is advantageous.

Also, the formulations disclosed in IN201741041173 primarily use monosaccharides in combination with sugar alcohols for the preparation of a stable pharmaceutical composition of trabectedin. Alternatively, also amino acids may be used as excipient. Exemplified is a formulation comprising trabectedin and L-arginine in a ratio of 1:400.

The inventors surprisingly found that even low amounts of amino acids stabilize trabectedin compositions without generating trabectedin degradation products. In this context, the term "stability" is understood to mean the definition developed by the Working Group for Pharmaceutical Process Engineering (APV), according to which "stability" means the quality of the medicinal product as specified until the end of the term specified by the manufacturer. The quality of the drug is determined by the active substance content and the purity, the sensorially perceptible, physicochemical and microbiological properties, whereby the active substance content should not fall below 90% of the declared value by the end of the term.

The formulation produced according to the invention has a stability (or in other words shelf life or running time) of at least 3 months, preferably of at least 6 months, more preferably of at least 12 months, even more preferably of at least 24 months at a temperature of 2 to 8 ° C. and most preferably of at least 36 months, whereby the trabectedin content by the end of the term does not fall below 90%, preferably 95%, of the trabectedin originally used.

The term "amino acid" refers to natural amino acids, including proteinogenic and non-proteinogenic amino acids, synthetic amino acids, and amino acid analogues, all in their D and L stereoisomers if their structure allows such stereoisomeric forms. Amino acids include non-polar, polar, basic and acidic amino acids. Non-polar proteinogenic amino acids are glycine (Gly), alanine (Ala), valine (Val), leucine (Leu), isoleucine (Ile), proline (Pro), tryptophan (Trp), phenylalanine (Phe) and methionine (Met). Polar proteinogenic amino acids include serine (Ser), threonine (Thr), tyrosine (Tyr), asparagine (Asn), glutamine (Gln) and cysteine (Cys). Basic proteinogenic amino acids include arginine (Arg), histidine (His) and lysine (Lys). Acidic proteinogenic amino acids include aspartic acid (Asp) and glutamic acid (Glu). Synthetic amino acids include acetylcysteine. Non-proteinogenic amino acids include citrulline.

The amino acid may be a non-polar, polar, basic or acidic amino acid, or any combination thereof. According to one embodiment, the amino acid is selected from the group of non-polar proteinogenic amino acids, specifically L-phenylalanine or L-isoleucine, or any combination thereof.

The ratio of trabectedin to amino acid in embodiments described herein is determined according to the solubility of the amino acid and, when the formulation is freeze-dried, also according to the freeze-dryability of the amino acid. The weight ratio (w/w) of trabectedin to amino acid (w/w) can be about 1:50, 1:60, 1:70, 1:80, 1:90, 1:100, wherein said amino acid is L-arginine. In some embodiments the weight ratio is in the range of 1:50 to 1:100. In a specific embodiment, the weight ratio is about 1:70.

The term "about" as used herein refers to values in the range of +/- 10% of the recited value.

In a specific aspect, the composition described herein further comprises a buffering agent. The buffering agent allows to maintain the pH of the system in a range which favors the stability of trabectedin. Typically, the pH will be in the range of pH 2 to pH 5. Suitable buffers are for example citrate buffer, phosphate buffer, citrate/phosphate buffer, lactate buffer, ascorbate buffer, tartaric/citrate buffer, bicarbonate/hydrochloric acid buffer, acetate or acetic acid buffer, succinate buffer, glycine/hydrochloric acid buffer. In some embodiments, buffers based on amino acids may be employed such as buffers based on amino acids already present in the composition. Mixtures of said buffer agents may also be used.

According to one embodiment, the buffering agent is selected from the group consisting of citric acid, phosphoric acid, acetic acid, basic amino acid and sodium hydroxide, or any mixture thereof. In a further embodiment, the buffering agent is a mixture of citric acid and phosphoric acid (citrate/phosphate buffer). Specifically, a citrate/phosphate buffer may further contain a basic amino acid, such as L-arginine, as buffering agent. Further, sodium hydroxide may be used for pH adjustment.

Other components may be included in the composition, for example a complexing agent such as ethylenediaminetetraacetic acid (EDTA); an antioxidant such as monothioglycerol; a surface-active agent; mannitol; and ascorbic acid. Examples for surface-active agents include polysorbate, polyoxyethylene (20) sorbitan monooleate or polyoxyl stearate, phospholipids, such as a lecithin; polyoxyethylene-polyoxypropylene copolymers, such as a Pluronic surfactant; polyoxyethylene esters of 12-hydroxysteraric acid, such as a Solutol surfactant; ethoxylates of cholesterol, such as diacyl glycerol, dialkyl glycerol; bile salts, such as sodium cholate, sodium deoxycholate; sucrose esters, as sucrose monolaurate, sucrose monooleate; polyvinyl pyrrolidone (PVP); or polyvinyl alcohol (PVA).

The composition described herein may be in the form of a lyophilized formulation. The term "lyophilized formulation" as used herein refers to a formulation prepared by lyophilization (freeze drying) of a mixture comprising a pharmaceutically effective amount of trabectedin. The inventors surprisingly found that the use of one or more amino acids as bulking agent in the lyophilization process improves the storage conditions and allows long term storage of the lyophilized formulation in a wide temperature range, including refrigeration conditions and room temperature.

The present invention therefore provides a lyophilized formulation comprising trabectedin, at least one amino acid, a buffering agent, and optionally a bulking agent such as mannitol. The amino acid may act as a bulking agent.

The term "bulking agent" as used herein refers to a compound which adds mass to a lyophilized (freeze dried) mixture and contributes to the physical structure of the lyophilized cake (e.g., facilitates the production of an essentially uniform lyophilized cake which maintains an open pore structure). In addition to providing a pharmaceutically elegant cake, bulking agents may also impart useful qualities in regard to modifying the collapse temperature, providing freeze-thaw protection, and enhancing the stability over long-term storage. Exemplary bulking agents include mannitol, glycine, lactose, sucrose, dextrose and hydroxyethyl starch. Bulking agents may be crystalline (such as glycine, or mannitol,) or amorphous (such as dextran, or hydroxyethyl starch).

In a specific embodiment, the amino acid in the lyophilized formulation described herein is a non-polar, polar, basic or acidic amino acid, or any combination thereof. The amino acid may be a non-polar amino acid such as L-phenylalanine or L-isoleucine, or a basic amino acid, e.g., L-arginine, L-lysine or L-histidine, or any combination thereof. Specifically, the amino acid is L-arginine. In one aspect, L-arginine may be combined with one or more further amino acids such as L-phenylalanine or L-isoleucine. In another embodiment, the amino acid is citrulline. In yet another embodiment the amino acid is acetylcysteine.

In one embodiment, the amino acid in said lyophilized formulation is L-arginine and the buffering agent is a mixture of citric acid and phosphoric acid (citrate/phosphate buffer). The amino acid may together with a base act additionally also as a buffering agent.

The lyophilized formulation according to the present invention can be prepared by freeze-drying a composition described herein in the form of a pre-lyophilization solution. Described herein is a method of preparing a lyophilized formulation comprising freeze-drying the composition in the form of a pre-lyophilization solution, wherein the pre-lyophilization solution comprises trabectedin at a concentration of 0.1 to 1 mg/mL, L-arginine at a concentration of 10 to 30 mg/mL, citric acid at a concentration of 0.5 to 4 mg/mL, and phosphoric acid at a concentration of 5 to 20 mg/mL. The pH of the pre-lyophilization solution is in the range of pH 2 to pH 5, specifically in the range between pH 4 to pH 5, more specifically the pH is 4.8. The method of preparing a lyophilized formulation may further involve adjusting the pH to said desired value, e.g., by adding phosphoric acid and/or sodium hydroxide.

In one aspect, the lyophilized formulation is provided in a vial. According to one embodiment, the vial is a molded glass vial or a tubing glass vial. However, the present invention is not limited by specific container forms or designs, as long as the container is acceptable for its intended use and standards therefore.

The lyophilized formulation is usually presented in a vial which contains a specified amount of trabectedin. E.g., the amount of trabectedin in the vial is 0,25 mg or 1 mg. To provide a vial containing the lyophilized formulation, the pre-lyophilization solution is added to the vial and freeze-dried. The volume of the pre-lyophilization added to the vial is in the range of 1 mL to 5 mL, or 1 to 4 mL.

In one embodiment, the vial contains 0.25 mg trabectedin, and 17.4 mg L-arginine, and 0.5 to 4 mg citric acid, e.g., 1.9 mg citric acid, and 5 to 20 mg phosphoric acid, or 10 to 15 mg phosphoric acid.

Providing the lyophilized formulation in a vial allows simple transport and handling, and a straightforward reconstitution to a formulation which can be readily administered to a patient in need thereof.

The lyophilized formulation can be reconstituted and diluted to give a composition in the form of a solution ready for intravenous injection.

The terms "reconstitute" or "reconstitution" as used herein refer to the process through which the lyophilized formulation is converted to a liquid form through the addition of, and mixing with, a pharmaceutically acceptable aqueous reconstitution solution, such as water for injection, sodium chloride solution or glucose solution.

The invention described herein provides an intravenous infusion comprising trabectedin, an amino acid, a buffering agent and water for injection. The invention further provides a method of preparing an intravenous infusion comprising providing a lyophilized formulation, reconstituting said lyophilized formulation in an aqueous system, and diluting said reconstituted formulation to a suitable concentration for intravenous infusion with an aqueous infusion medium.

The actual amounts of reconstitution solution are not limiting features of embodiments of this invention. By way of illustrations, but not as limitations, embodiments of lyophilized formulations according to this invention are reconstituted with a volume of water. Most of such volumes do not exceed about 20 mL, with preferred volumes being in the range from about 1 mL to about 15 mL, or in the range from about 1 mL to about 10 mL, or about 5 mL. The reconstituted solution in such embodiments contains a concentration of trabectedin of about 0.05 mg/mL, about 0.1 mg/mL, or about 0.15 mg/mL.

Reconstituted solutions can further be diluted if so desired. This further dilution may be carried out with an aqueous infusion medium which is usually 0.9 % sodium chloride or 5 % glucose. The reconstituted solution will be diluted depending on the concentration in the reconstituted solution and the desired concentration in the diluted solution.

In one aspect, the intravenous infusion is used in the treatment of cancer. In one aspect, the cancer is sarcoma, selected from the group of leiomyosarcoma, liposarcoma, osteosarcoma; ovarian cancer, breast cancer, melanoma, colorectal cancer, mesothelioma, renal cancer, endometrial cancer and lung cancer, or any combination thereof, and conditions with a plurality of such forms of cancer. It is understood that "treatment" in this context refers to an action that leads to an amelioration of the cancer condition(s). Embodiments of formulations according to the invention can also be used in the treatment of refractory cancer conditions that have not responded favorably to other treatments. In one embodiment, the intravenous infusion is used for the treatment of adult patients with advanced soft tissue sarcoma.

Trabectedin can be used in combination with another drug. For example, it can be administered with another antitumor drug. Examples of such other drugs include doxorubicin, cisplatin, paclitaxel, carboplatin, pegylated liposomal doxorubicin, docetaxel, capecitabine, and gemcitabine. Drugs with other modes of action can be used, including dexamethasone. Administration of the other drug can be before, during or after administration of trabectedin.

In another embodiment, the intravenous infusion described herein is used for the treatment of patients with relapsed platinum-sensitive ovarian cancer, wherein the treatment is in combination with pegylated liposomal doxorubicin (PLD).

Specifically, the intravenous infusion described herein is administered intravenously on a cyclic basis, e.g., for 1 to 20 cycles. The cycle includes a phase of infusing the trabectedin infusion, and usually also a phase of not infusing trabectedin. Typically, the cycle is worked out in weeks, and thus the cycle normally comprises one or more weeks of a trabectedin infusion phase, and one or more weeks to complete the cycle. A cycle of 3 weeks is preferred, but alternatively it can be from 1 to 6 weeks. The infusion phase can itself be a single administration in each cycle of e.g., 1 to 72 h, more usually of about 1, 3 or 24 h; or an infusion on a daily basis in the infusion phase of the cycle for 1 to 5 h, especially 1 or 3 h; or an infusion on a weekly basis in the infusion phase of the cycle for 1 to 3 h, especially 2 or 3 h. A single administration at the start of each cycle is preferred. Specifically, the infusion time is about 1, 3 or 24 h.

Exemplary dosing protocols of intravenous trabectedin formulations are e.g., described in WO2006046079. Such dosing protocols include:
a) about 1.5 mg/m² body surface area, administered as an intravenous infusion over 24 h with a three-week interval between cycles;
b) about 1.3 mg/m² body surface area, administered as an intravenous infusion over 3 h with a three-week interval between cycles;
c) about 0.580 mg/m2 body surface area, administered weekly as an intravenous infusion over 3 h during 3 weeks and 1 week rest.

### Examples

The Examples which follow are set forth to aid in the understanding of the invention but are not intended to, and should not be construed to limit the scope of the invention in any way. The Examples do not include detailed descriptions of conventional methods, e.g., quantification by high-pressure liquid chromatography (HPLC). Such methods are well known to those of ordinary skill in the art.

### Example 1 - Formulations Variants

Formulation variants for trabectedin as depicted in Table 1 were provided. Different bulking agents were used. For example, "0.1 M Arg" refers to L-arginine at a concentration of 0.1 mol/L.

**Table 1: Formulations Variants**

| **#** | **Bulking Agent** | **Buffer** | **pH Adjustment** | **pH** |
|---|---|---|---|---|
| 1 | 0.1 M Arg, 0.1 M Phe | 10 mM citric acid | H₃PO₄ | 4.8 |
| 2 | 0.1 M Arg, 0.1 M Ile | 10 mM citric acid | H₃PO₄ | 4.8 |
| 3 | 0.1 M Arg | 10 mM citric acid | L-aspartic acid | 4.8 |
| 4 | 0.1 M Arg | 10 mM citric acid | H₃PO₄ | 4.8 |
| 5 | 0.1 M Arg | 10 mM citric acid | H₃PO₄ | 3.8 |
| 6 | 0.1 M Arg | 10 mM citric acid | H₃PO₄ | 3.4 |
| 7 | 0.1 M Arg | 10 mM citric acid | H₃PO₄ | 3.0 |
| 8 | 0.1 M Arg, 0.1 M Phe | 10 mM citric acid | NaOH | 3.0 |
| 9 | 0.1 M Arg, 0.1 M Phe | 10 mM citric acid | NaOH | 3.8 |

### Example 2 - Short Term Stability of Pre-Lyophilization Solutions

In total, 9 pre-lyophilization solutions were prepared. The formulation variants were compounded by pre-weighing approximately 10 mg trabectedin in a 50 mL glass vessel and adding the calculated amount of solvent to reach a target concentration of trabectedin of 0.25 mg/mL. The pre-lyophilization solutions were mixed on a roller mixer for approximately 30 minutes at room temperature (22° C). The solutions were filtered using 0.2 µm syringe filters (Millipore, Fluorodyne^{®}II membrane) and filled (1 mL per vial) in cleaned and depyrogenized 10R glass vials.

The stability of the liquid formulations of trabectedin (0.25 mg/mL) was tested in a short-term (0h and 24 h) stability experiment at 2-8 ° C and 25 ° C. Some variance in the trabectedin content was observed by RP-HPLC analysis (for parameters see Table 4) between different formulation variants, however the differences between stressed (24 h, at 2-8 ° C or 25 ° C) and unstressed (0 h) samples was marginal (see Table 2). Table 2 shows the results of the trabectedin stability assay. The content of trabectedin as determined by RP-HPLC (detection at 285 nm) is given in % of the reference. Purity is analyzed as relative peak area. The amounts of the impurities listed in Table 2 were determined. Amounts were determined as relative peak area in %. Most impurities were detected only in marginal amounts (Table 2). A temperature dependence of trabectedin stability could be observed. Low pH variants seemed significantly more stable compared to their high pH equivalents.

**Table 2: Results of stability tests: content of trabectedin and impurities as determined by HPLC**

| | Trabected in | | RRT 0.94 | RR T 0.7 3 | RRT 1.12 | RRT 1.20 | RR T 1.4 3 | RR T 1.6 9 | RRT 1.32 | RR T 0.2 7 | RR T 0.3 | RR T 0.7 8 | RR T 1.42 |
|---|---|---|---|---|---|---|---|---|---|---|---|---|---|
| # | Ass ay | Rel. Are a | Rel. Are a | Rel. Are a | Rel. Are a | Rel. Are a | Rel. Are a | Rel. Are a | Rel. Are a | Rel. Are a | Rel. Are a | Rel. Are a | Rel. Are a |
| | % | % | % | % | % | % | % | % | % | % | % | % | % |
| #1 0 h | 100 .6 | 99. 83 | < 0.1 | < 0.1 | < 0.1 | < 0.1 | < 0.1 | < 0.1 | 0.17 | < 0.1 | 0.98 | < 0.1 | < 0.1 |
| #1 | 99. 8 | 99. 80 | < 0.1 | < 0.1 | < 0.1 | < 0.1 | < 0.1 | < 0.1 | 0.20 | < 0.1 | 1.10 | < 0.1 | < 0.1 |
| 5°C 24 h | | | | | | | | | | | | | |
| #1 25°C 24 h | 100 .1 | 99. 57 | < 0.1 | < 0.1 | < 0.1 | < 0.1 | < 0.1 | < 0.1 | 0.43 | < 0.1 | 2.31 | < 0.1 | < 0.1 |
| #2 0 h | 97. 9 | 99. 89 | < 0.1 | < 0.1 | < 0.1 | < 0.1 | < 0.1 | < 0.1 | 0.11 | < 0.1 | 0.66 | < 0.1 | < 0.1 |
| #2 5°C 24 h | 97. 3 | 99. 89 | < 0.1 | < 0.1 | < 0.1 | < 0.1 | < 0.1 | < 0.1 | 0.11 | < 0.1 | 0.71 | < 0.1 | < 0.1 |
| #2 25°C 24 h | 97. 9 | 99. 75 | < 0.1 | < 0.1 | < 0.1 | < 0.1 | < 0.1 | < 0.1 | 0.25 | < 0.1 | 1.44 | < 0.1 | < 0.1 |
| #3 0 h | 97. 6 | 99. 85 | < 0.1 | < 0.1 | < 0.1 | < 0.1 | < 0.1 | < 0.1 | 0.15 | < 0.1 | 0.89 | < 0.1 | < 0.1 |
| #3 5°C 24 h | 97. 3 | 99. 83 | < 0.1 | < 0.1 | < 0.1 | < 0.1 | < 0.1 | < 0.1 | 0.17 | < 0.1 | 0.99 | < 0.1 | < 0.1 |
| #3 25°C 24 h | 97. 6 | 99. 62 | < 0.1 | < 0.1 | < 0.1 | < 0.1 | < 0.1 | < 0.1 | 0.38 | < 0.1 | 2.22 | < 0.1 | < 0.1 |
| #4 0 h | 94. 7 | 99. 89 | < 0.1 | < 0.1 | < 0.1 | < 0.1 | < 0.1 | < 0.1 | 0.11 | < 0.1 | 0.66 | < 0.1 | < 0.1 |
| #4 5°C 24 h | 94. 4 | 99. 88 | < 0.1 | < 0.1 | < 0.1 | < 0.1 | < 0.1 | < 0.1 | 0.12 | < 0.1 | 0.72 | < 0.1 | < 0.1 |
| #4 25°C 24 h | 94. 9 | 99. 70 | < 0.1 | < 0.1 | < 0.1 | < 0.1 | < 0.1 | < 0.1 | 0.30 | < 0.1 | 1.65 | < 0.1 | < 0.1 |
| #8 0 h | 100 .9 | 100 | < 0.1 | < 0.1 | < 0.1 | < 0.1 | < 0.1 | < 0.1 | < 0.1 | 0.17 | < 0.1 | < 0.1 | < 0.1 |
| #8 5°C 24 h | 94. 6 | 99. 67 | < 0.1 | < 0.1 | < 0.1 | 0.33 | < 0.1 | < 0.1 | < 0.1 | 0.16 | 0.47 | < 0.1 | 0.31 |
| #8 25°C 24 h | 95. 6 | 99. 29 | 0.13 | 0.23 | < 0.1 | 0.35 | < 0.1 | < 0.1 | < 0.1 | 0.3 | 1.13 | < 0.1 | 0.79 |
| #9 0 h | 98. 0 | 100 | < 0.1 | < 0.1 | < 0.1 | < 0.1 | < 0.1 | < 0.1 | < 0.1 | 0.47 | < 0.1 | < 0.1 | < 0.1 |
| #9 5°C 24 h | 89. 6 | 98. 98 | 0.17 | 0.11 | < 0.1 | 0.61 | < 0.1 | < 0.1 | 0.13 | 0.71 | 2.07 | < 0.1 | 1.53 |
| #9 25°C 24 h | 90. 9 | 98. 67 | 0.19 | 0.21 | < 0.1 | 0.66 | < 0.1 | < 0.1 | 0.28 | 1.36 | 3.06 | < 0.1 | 2.35 |

### Example 3 - Lyophilization

Filled vials were stoppered in lyophilization position loaded in stainless steel trays and wrapped in LyoProtect^{®} bags (bags with steam permeable PTFE membrane, to prevent contamination of the freeze dryer). Two pilot freeze dryers (Hof Sonderanlagenbau, Lohra, Germany) were used (GT2, 0.36 m² shelf area and GF3, 0.25 m² shelf area). As several different formulation variants were freeze dried in one lyophilization run (run 1: formulation variants #1-#4, run 2: formulation variants #8-#9), generic conservative lyophilization parameters were selected (Table 3).

**Table 3: Parameters of feasibility lyophilization**

| **Step** | | **Shelf temperature** | **Ice condenser temperature** | **Pressure** | **Time step** |
|---|---|---|---|---|---|
| | Description | [° C] | [° C] | [mbar] | [h:min] |
| 1 | Loading | 20 | - | 1000 | 00:01 |
| 2 | Freezing ramp | -45 | - | 1000 | 01:05 |
| 3 | Freezing | -45 | - | 1000 | 01:00 |
| 4 | Annealing ramp | -20 | - | 1000 | 00:25 |
| 5 | Annealing | -20 | - | 1000 | 02:00 |
| 6 | Freezing ramp | -45 | - | 1000 | 00:25 |
| 7 | Freezing | -45 | - | 1000 | 02:00 |
| 8 | Vacuum adjustment | -45 | -70 | 0.05 | 00:30 |
| 9 | Primary Drying Ramp | -10 | -70 | 0.05 | 01:00 |
| 10 | Primary Drying | -10 | -70 | 0.05 | xx:xx* |
| 11 | Secondary drying | 30 | -70 | 0.05 | 04:00 |
| 12 | Secondary drying | 30 | -70 | 0.05 | 10:00 |

| | | | | | |
|---|---|---|---|---|---|
| * Primary Drying was run overnight without operator attendance. The recorded drying times ranged from 14 to 16 hours. It is noted that for process development purposes a much shorter time for Primary Drying would probably be sufficient. | | | | | |

All formulation variants were suitable for lyophilization. Most variants showed some increase in impurities (especially RRT 1.32 (A285 nm) during lyophilization, indicating a sensitivity of trabectedin.

In order to differentiate the formulation variants further, the stability of lyophilized samples of all formulation variants #1-4 and #8-9 was evaluated in an accelerated stability study.

### Example 4 -Short Term Stability Study of Lyophilized Samples

Samples obtained in the feasibility lyophilization were put on stability testing at three temperatures: 2-8° C, 25 ° C and 40 ° C. Samples were analyzed immediately after lyophilization (T0), after one month (T1), two months (T2), three months (T3) and after 6 months (T6) storage period with respect to visual appearance, reconstitution speed, degradation profile by RP-HPLC and residual water content using a generic Karl Fischer oven method (only T0).

Samples of each formulation variant were stored and analyzed. All formulation variants formed solid cakes which remained visually stable during the testing period. Reconstitution in 1 mL water for injection was fast and spontaneous within 10 seconds for all variants at all time points.

### Content and purity of lyophilizates

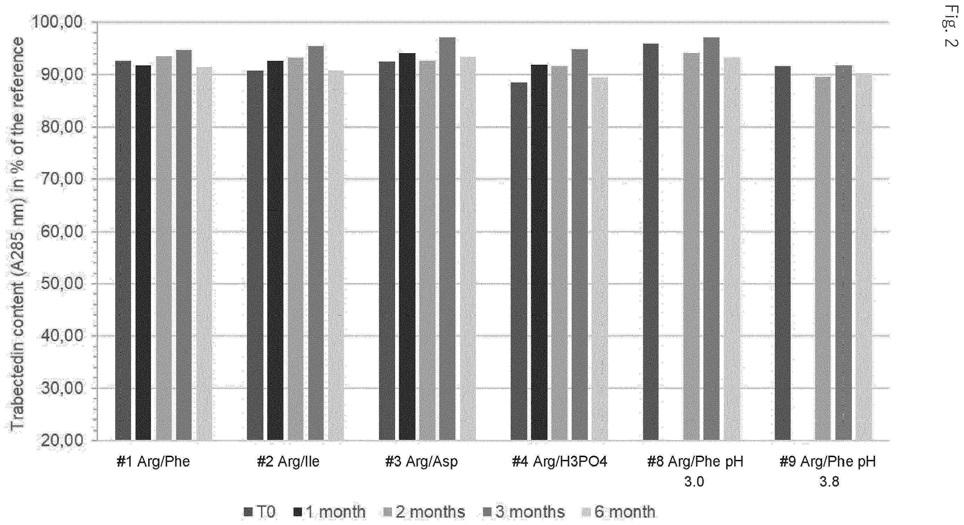

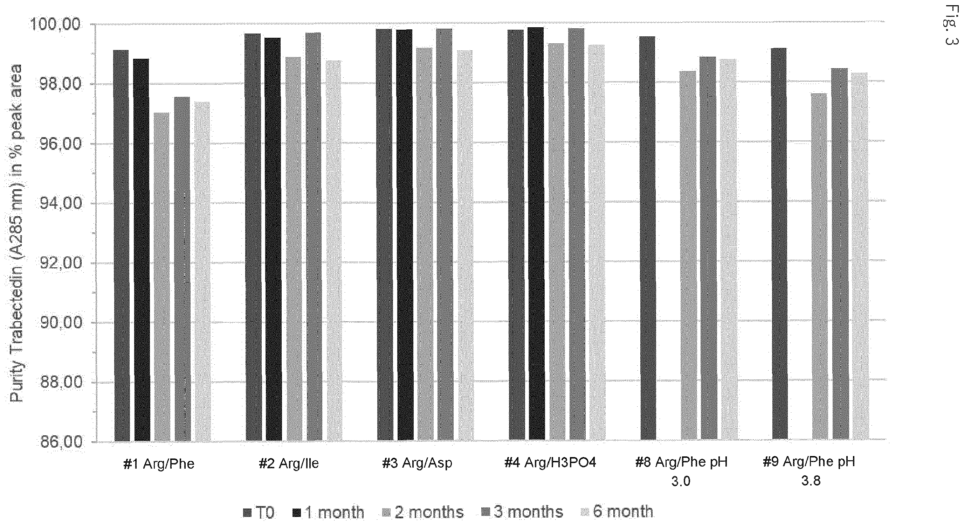

Reconstituted lyophilizates were analyzed by RP-HPLC (for parameters see Table 4), to determine the trabectedin content and impurity profile. Results for storage at 2-8° C are summarized in Fig. 2 and Fig. 3.

**Table 4: Parameters for HPLC measurements**

| **Parameter** | **Value** | | |
|---|---|---|---|
| Column temperature | 15 °C | | |
| Sampler temperature | 5 °C | | |
| Wavelength | DAD: 285 nm, 255 nm* | | |
| Pump flow | 1.2 mL/min | | |
| Run time | 60 min | | |
| Column | Phenyl 250 x 4,6 mm; 5 µm; 80 A (ZORBAX SB-Phenyl, Agilent - pressure limit 400 bar) | | |
| Buffer A | A: 1 g Ammonium formiate + 1 mL formic acid in 1 L water | | |
| Buffer B | B: AcN | | |
| Gradient | 0 min: | 85 % A | 15 % B |
| | 30 min: | 73 % A | 27 % B |
| | 50 min: | 35 % A | 65 % B |
| | 52 min: | 85 % A | 15% B |
| | 60 min: | 85 % A | 15 % B |
| Injection volume | 20 µL | | |
| Concentration of standard/sample | 0.25 mg/mL | | |
| Diluent | Methanol | | |

The formulation with L-arginine in citric acid comprising H₃PO₄ for pH adjustment to pH 4.8 (formulation #4, see Table 1 and Table 5) shows very good stability results, even at 40° C storage temperature.

**Table 5: Composition of formulation #4 (100 mM L-arginine)**

| **Component** | **Function** | **Amount in mg/vial** |
|---|---|---|
| Trabectedin | Active ingredient | 0.25 |
| Citric Acid | Buffering agent | 1.92 |
| L-Arginine | Bulking agent | 17.42 |
| Phosphoric Acid (85 % w/w in water) | Buffering agent/pH adjustment | Depending on desired pH, e.g., 10 to 15 mg required to achieve pH 3 |
| NaOH | Buffering agent/pH adjustment | Dependent on desired pH; used for pH-correction |

### Example 5 - 3 Months Stability Studies

Three different formulation variants (arginine-phosphate pH 3.0, pH 3.4, and pH 3.8, see Table 1 and 5) were prepared, lyophilized and analyzed concerning assay and impurity profile over time.

### Experimental Details

For the stability studies, the lyophilized samples of trabectedin formulation variants were stored at 2-8° C, 25 ° C and 30° C. Placebo solutions without trabectedin were stored at 25° C and are utilized as reference samples for HPLC analysis. The analysis time points are after 1 month, 3 months (see Table 6 and 7).

**Table 6: Impurities detected at 285 nm after 3 months storage at 2-8 ° C**

| **Impurities** | **# 7 (Arg pH 3.0)** | **#6 (Arg pH 3.4)** | **#5 (Arg pH 3.8)** |
|---|---|---|---|
| RRT 0.12 [%] | 0.16 | 0.29 | 0.36 |
| RRT 1.16 [%] | 0.23 | 0.46 | 0.49 |
| RRT 1.28 [%] | 0.10 | 0.15 | 0.16 |
| RRT 0.73 [%] | 0.11 | 0.12 | 0.15 |

**Table 7: Impurities detected at 285 nm after 3 months storage at 25 ° C**

| **Impurities** | **# 7 (Arg pH 3.0)** | **#6 (Arg pH 3.4)** | **#5 (Arg pH 3.8)** |
|---|---|---|---|
| RRT 0.12 [%] | 0.42 | 0.52 | 0.60 |
| RRT 1.16 [%] | 0.22 | 0.42 | 0.47 |
| RRT 1.28 [%] | 0.12 | 0.19 | 0.20 |
| RRT 0.73 [%] | 0.64 | 0.44 | 0.48 |

### Example 6 - 6 Months Stability Studies

### Compounding of the lyophilization solution

12 different formulation variants (see Table 8) were prepared, lyophilized and analyzed concerning trabectedin content and purity profile over time. Each formulation contains 0.25 mg/mL trabectedin before lyophilization.

**Table 8: Formulations Variants**

| # | **Bulking Agent** | **Buffer** | **pH Adjustment** | **pH** |
|---|---|---|---|---|
| 1 | 0.1 M Arg | 10 mM citric acid | H₃PO₄ | 4.0 |
| 2 | 0.1 M Arg | 10 mM citric acid | H₃PO₄ | 4.0 |
| | 35 mg mannitol (w/w) | | | |
| 3 | 0.1 M Arg | 10 mM Na acetate | H₃PO₄ | 4.0 |
| 4 | 0.1 M L-acetyl cysteine | 10 mM citric acid | NaOH | 4.0 |
| 5 | 0.1 M Arg | 10 mM citric acid | NaOH | 4.0 |
| | 0.2 % (w/V) L-ascorbic acid | | | |
| 6 | 0.1 M Valin | 10 mM citric acid | H₃PO₄ | 4.0 |
| 7 | 0.1 M L-aspartic acid | - | lysine | 4.0 |
| 8 | 0.1 M L-citrulline | 10 mM citric acid | NaOH | 4.0 |
| 9 | 0.1 M Arg | 10 mM citric acid | H₃PO₄ | 4.0 |
| | 10 mM monothioglycerol | | | |
| 10 | 0.1 M Arg | 10 mM citric acid | H₃PO₄ | 4.0 |
| | 0.2 % (w/V) L-methionine | | | |
| 11 | 0.1 M Arg | 10 mM citric acid | H₃PO₄ | 4.0 |
| | 0.2 % (w/V) Tween 20 | | | |
| 12 | 0.1 M Arg | 10 mM citric acid | H₃PO₄ | 4.0 |
| | 0.1 % (w/V) EDTA | | | |

The selected formulation variants were compounded by pre-weighing approximately 12.5 mg trabectedin in a 50 mL glass vial and adding the calculated amount of solvent to reach a target concentration of trabectedin of 0.25 mg/mL.

Each of the 12 formulation variants (API-free buffer) was prepared by individually weighing the corresponding substances into a beaker, dissolution in 90 % of the designated volume of purified water, pH adjustment with sodium hydroxide solution (30 %) or hydrochloric acid solution (25 %) for variant #7, and ortho-phosphoric acid (85 %) for other variants, and subsequent addition of purified water to the final volume. The calculated amount of solvent (buffer) was then added to pre-weighed trabectedin in a 50 mL glass vial to reach a target concentration of trabectedin of 0.25 mg/mL. The buffer volume for dilution has been adapted to the actual weighed in masses for 0.25 mg/mL for each variant. The solution was stirred on a magnetic stirrer for approximately 30 minutes at room temperature (22 ° C) until complete dissolution. The lyo solution was filtered using 0.2 µm syringe filters (Millipore, Fluorodyne^{®}II membrane) and filled (1 mL per vial) in cleaned and depyrogenized 10R glass vials.

### Lyophilization

Filled vials were partially stoppered in lyophilization position, loaded in stainless steel trays and wrapped and sealed in LyoProtect^{®} bags (bags with steam permeable PTFE membrane, to prevent contamination of the freeze dryer). Vials were loaded into a freeze dryer and lyophilized.

After lyophilization vials were vented to 750 mbar with nitrogen and vials were closed. After unloading vials were crimp sealed and decontaminated with 70 % isopropanol/water.

### Storage

Lyophilized samples were stored at 25 ° C for up to six months and analyzed immediately after lyophilization (T0), after one month (T1m), three months (T3m) and 6 months (T6m) storage period with respect to
▪ visual appearance,
▪ reconstitution behavior;
▪ clarity of solution (nephelometric turbidity);
▪ pH measurement after reconstitution;
▪ degradation profile by RP-HPLC; and
▪ residual water content using a generic Karl Fischer oven method (only T0).

### Reconstitution

Stoppers were removed from the vials and the lyophilizates were reconstituted with 5.0 mL purified water using a pipette; reconstitution speed and behavior were monitored.

### Residual water content analysis by generic Karl-Fischer oven method

The residual moisture of the samples was determined via Karl Fischer titration using a 756 Karl Fischer coulometer equipped with a 774 oven sample processor (Metrohm).

For each measurement, one lyophilizate of each sample (≈ 20-50 mg) was transferred in a Karl-Fischer vial. The exact weight was documented. The vial was sealed with a crimp cap and transferred into the oven of the Karl Fischer coulometer which was heated to 110 ° C. The septum of the crimp cap was penetrated by an injection needle, and the generated water vapor was directly transferred into the titration chamber via dry nitrogen. Empty glass vials were used as blank correction. Samples were analyzed in duplicate.

### pH measurement

The pH of the samples was measured directly in the vial after reconstitution (5 mL purified water) using a common pH-meter. pH meter: SevenMulti, Mettler Toledo with micro electrode. The pH-meter was calibrated prior use at pH 4.0, pH 7.0 and pH 9.0.

### Content and purity by RP-HPLC

RP-HPLC was conducted according to Table 4.

Detection of impurities was done at two different detector wave lengths (285 nm and 255 nm) to cover all possible impurities. It was observed that some impurities showed higher absorption at one of the wave lengths than at the other. Therefore, the evaluation approach at two different wavelengths supports the complete coverage of all relevant impurities.

### Sample preparation:

For RP-HPLC analysis the lyophilizate was reconstituted with 1 mL purified water. No further sample dilution for HPLC analysis was required.

### Content and Purity of Lyophilizates

Reconstituted lyophilizates were analyzed by RP-HPLC to determine the trabectedin content and purity profile. The results were summarized in Fig. 13 and Fig. 14. Fig. 13 shows the content of trabectedin at T0, T1m, T3m and T6m, measured by RP-HPLC in % of the reference. All samples show a stable trabectedin content over the time. Fig. 14 shows the purity of the samples as relative peak area of trabectedin analyzed by RP-HPCL. All samples show a high purity profile over the time.

### Impurity Profile of The Samples

The samples were stored at 25° C and tested after 1, 3 and 6 months. 25° C is considered accelerated testing conditions which allows evaluation of stability of formulations. The long-term storage conditions would most likely be refrigerated storage (2 - 8° C).

The impurities of the samples were analyzed and results are presented in following tables.

**Table 9 - impurity profile of #1 (0.1 M Arg, 10 mM citric acid, H₃PO₄)**

| | **Relative peak area of impurity [%]** | | | |
|---|---|---|---|---|
| **Impurities at** 285 nm | **T0** | **T1m** | **T3m** | **T6m** |
| RRT 0.11 | <0.1 | <0.1 | <0.1 | 0.11 |
| RRT 0.12 | <0.1 | <0.1 | 0.43 | 0.51 |
| Et-701 | <0.1 | 0.13 | 0.26 | 0.27 |
| Et-759B | <0.1 | <0.1 | <0.1 | 0.10 |
| RRT 1.24 | <0.1 | <0.1 | <0.1 | 0.15 |
| RRT 1.35 | <0.1 | <0.1 | 0.26 | 0.11 |
| RRT 1.84 | <0.1 | <0.1 | <0.1 | 0.40 |
| **TOTAL 285 nm** | **0.00** | **0.13** | **0.95** | **1.65** |

| **Impurities at 255 nm** | **T0** | **T1m** | **T3m** | **T6m** |
|---|---|---|---|---|
| RRT 0.27 | 0.18 | 0.39 | 1.08 | 0.42 |
| RRT 0.31 | 0.30 | 0.36 | 1.10 | 0.35 |
| RRT 0.76 | 0.13 | 0.30 | 0.89 | 0.36 |
| RRT 1.38 | <0.1 | 0.34 | 1.24 | 0.27 |
| RRT 1.46 | <0.1 | 0.19 | <0.1 | 0.49 |
| RRT 1.65 | <0.1 | 0.23 | <0.1 | 0.64 |
| **TOTAL 255nm** | **0.61** | **1.81** | **4.31** | **2.53** |

| | | | | |
|---|---|---|---|---|
| means of two injections are reported if >0.1 % | | | | |

**Table 10 - impurity profile of #2 (0.1 M Arg, 35 mg/ml mannitol, 10 mM citric acid, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.11 | <0.1 | <0.1 | <0.1 | 0.13 |
| RRT 0.12 | <0.1 | <0.1 | 1.14 | 1.26 |
| RRT 0.17 | <0.1 | <0.1 | 0.23 | 0.17 |
| RRT 0.40 | <0.1 | <0.1 | 0.13 | 0.10 |
| RRT 0.43 | <0.1 | <0.1 | <0.1 | 0.14 |
| RRT 0.45 | <0.1 | 0.17 | 0.19 | 0.12 |
| Et-701 | 0.16 | 0.67 | 0.68 | 0.94 |
| Et-745 | <0.1 | 0.28 | 0.84 | 0.70 |
| RRT 1.24 | <0.1 | 1.14 | 2.26 | 3.27 |
| RRT 1.35 | <0.1 | <0.1 | <0.1 | 0.15 |
| Et-759A | <0.1 | 0.26 | 0.44 | 0.33 |
| RRT 1.51 | <0.1 | <0.1 | <0.1 | 0.13 |
| RRT 1.84 | <0.1 | <0.1 | <0.1 | 0.36 |
| **TOTAL** | **0.16** | **2.52** | **5.91** | **7.80** |

| **Impurities at 255 nm** | **T0** | **T1m** | **T3m** | **T6m** |
|---|---|---|---|---|
| RRT 0.27 | 0.20 | 1.28 | 2.84 | 2.84 |
| RRT 0.31 | 0.35 | 0.53 | 1.31 | 0.58 |
| RRT 0.76 | 0.14 | 0.62 | 1.08 | 0.74 |
| RRT 1.23 | <0.1 | 0.25 | 0.48 | 0.98 |
| RRT 1.38 | <0.1 | 0.53 | 1.97 | 1.69 |
| RRT 1.46 | <0.1 | 0.11 | 0.16 | 0.29 |
| RRT 1.57 | <0.1 | <0.1 | <0.1 | 0.15 |
| RRT 1.65 | <0.1 | <0.1 | <0.1 | 0.31 |
| RRT 1.69 | <0.1 | 0.11 | <0.1 | 0.31 |
| **TOTAL** | **0.69** | **3.43** | **7.84** | **7.89** |

| | | | | |
|---|---|---|---|---|
| means of two injections are reported if >0.1 % | | | | |

**Table 11 - Impurity profile of #3 (0.1 M Arg, 10 mM Na acetate, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.11 | <0.1 | <0.1 | <0.1 | 0.13 |
| RRT 0.12 | <0.1 | <0.1 | 0.45 | 0.53 |
| Et-701 | <0.1 | 0.14 | 0.31 | 0.38 |
| RRT 1.24 | <0.1 | <0.1 | 0.27 | 0.16 |
| RRT 1.66 | <0.1 | <0.1 | <0.1 | 0.10 |
| RRT 1.84 | <0.1 | <0.1 | <0.1 | 0.36 |
| **TOTAL** | **0.00** | **0.14** | **1.03** | **1.66** |

| **Impurities at 255 nm** | **T0** | **T1m** | **T3m** | **T6m** |
|---|---|---|---|---|
| RRT 0.27 | 0.29 | 0.64 | 1.01 | 0.58 |
| RRT 0.31 | 0.32 | 0.53 | 1.14 | 0.44 |
| RRT 0.76 | 0.21 | 0.49 | 0.81 | 0.45 |
| RRT 1.38 | 0.16 | 0.71 | 1.05 | 0.43 |
| RRT 1.46 | <0.1 | <0.1 | <0.1 | 0.27 |
| RRT 1.57 | <0.1 | <0.1 | <0.1 | 0.11 |
| RRT 1.65 | <0.1 | <0.1 | <0.1 | 0.52 |
| RRT 1.69 | <0.1 | <0.1 | <0.1 | 0.23 |
| **TOTAL** | **0.98** | **2.37** | **4.01** | **3.03** |

| | | | | |
|---|---|---|---|---|
| means of two injections are reported if >0.1 % | | | | |

**Table 12 - impurity profile of #4 (0.1 M L-acetyl cysteine, 10 mM citric acid, NaOH)**

| | **Relative peak area of impurity [%]** | | | |
|---|---|---|---|---|
| **Impurities at 285 nm** | **T0** | **T1m** | **T3m** | **T6m** |
| RRT 0.08 | <0.1 | <0.1 | <0.1 | 2.40 |
| RRT 0.11 | <0.1 | <0.1 | 0.29 | 0.44 |
| RRT 0.12 | <0.1 | <0.1 | <0.1 | 0.40 |
| RRT 0.55 | <0.1 | <0.1 | 0.17 | <0.1 |
| Et-701 | 0.14 | 0.45 | 0.51 | 0.87 |
| RRT 0.96 | 0.84 | 0.80 | 0.82 | 0.69 |
| RRT 1.84 | <0.1 | <0.1 | <0.1 | 0.35 |
| **TOTAL** | **0.98** | **1.25** | **1.79** | **5.15** |

| **Impurities at 255 nm** | **T0** | **T1m** | **T3m** | **T6m** |
|---|---|---|---|---|
| RRT 0.31 | <0.1 | 0.18 | 0.32 | <0.1 |
| RRT 1.57 | <0.1 | <0.1 | <0.1 | 0.18 |
| RRT 1.69 | <0.1 | <0.1 | <0.1 | 0.11 |
| **TOTAL** | **0.00** | **0.18** | **0.32** | **0.29** |

| | | | | |
|---|---|---|---|---|
| means of two injections are reported if >0.1 % | | | | |

**Table 13 - impurity profile of #5 (0.1 M Arg, 0.2 % (w/V) L-ascorbic acid, 10 mM citric acid, NaOH)**

| | **Relative peak area of impurity [%]** | | | |
|---|---|---|---|---|
| **Impurities at 285 nm** | **T0** | **T1m** | **T3m** | **T6m** |
| RRT 0.10 | <0.1 | <0.1 | <0.1 | 0.76 |
| RRT 0.11 | <0.1 | <0.1 | 0.24 | 0.66 |
| RRT 0.12 | <0.1 | <0.1 | 0.18 | 0.25 |
| RRT 0.15 | <0.1 | <0.1 | <0.1 | 0.26 |
| RRT 0.45 | <0.1 | 0.18 | 0.22 | 0.21 |
| Et-701 | <0.1 | 0.19 | 0.32 | 0.32 |
| RRT 0.85 | <0.1 | <0.1 | 0.10 | <0.1 |
| RRT 1.84 | <0.1 | <0.1 | <0.1 | 0.31 |
| **TOTAL** | **0.00** | **0.37** | **1.06** | **2.77** |

| **Impurities at 255 nm** | **T0** | **T1m** | **T3m** | **T6m** |
|---|---|---|---|---|
| RRT 0.27 | 0.20 | 0.29 | 0.42 | 0.35 |
| RRT 0.76 | 0.12 | 0.18 | 0.30 | 0.22 |
| RRT 1.46 | <0.1 | <0.1 | <0.1 | 0.11 |
| RRT 1.57 | <0.1 | <0.1 | <0.1 | 0.12 |
| RRT 1.65 | <0.1 | <0.1 | 0.22 | 0.45 |
| **TOTAL** | **0.32** | **0.47** | **0.94** | **1.25** |

| | | | | |
|---|---|---|---|---|
| means of two injections are reported if >0.1 % | | | | |

**Table 14 - impurity profile of #6 (0.1 M Valin, 10 mM citric acid, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.11 | <0.1 | <0.1 | <0.1 | 0.10 |
| RRT 0.12 | <0.1 | <0.1 | 1.62 | 2.31 |
| RRT 0.43 | <0.1 | <0.1 | <0.1 | 0.14 |
| RRT 0.45 | <0.1 | 0.32 | 0.11 | 0.24 |
| Et-701 | 0.13 | 0.65 | 0.25 | 0.45 |
| RRT 0.77 | <0.1 | 0.32 | 1.12 | 0.92 |
| RRT 0.92 | <0.1 | <0.1 | <0.1 | 0.13 |
| Et-745 | <0.1 | 0.39 | 0.17 | 0.28 |
| Et-759B | <0.1 | <0.1 | 0.43 | 0.18 |
| RRT 1.24 | <0.1 | <0.1 | 0.27 | 0.20 |
| RRT 1.35 | <0.1 | <0.1 | 0.13 | 0.32 |
| Et-759A | <0.1 | 0.37 | <0.1 | 0.19 |
| RRT 1.51 | <0.1 | <0.1 | <0.1 | 0.21 |
| RRT 1.66 | <0.1 | <0.1 | <0.1 | 0.10 |
| RRT 1.84 | <0.1 | <0.1 | <0.1 | 0.33 |
| **TOTAL** | **0.13** | **2.05** | **4.10** | **6.10** |

| **Impurities at 255 nm** | **T0** | **T1m** | **T3m** | **T6m** |
|---|---|---|---|---|
| RRT 0.22 | <0.1 | <0.1 | 0.31 | 0.32 |
| RRT 0.27 | 0.76 | 3.85 | 9.56 | 10.94 |
| RRT 0.31 | <0.1 | 0.47 | 1.09 | 0.44 |
| RRT 0.76 | 0.59 | 2.91 | 6.07 | 7.84 |
| RRT 1.38 | <0.1 | 0.27 | 0.55 | 0.17 |
| RRT 1.46 | <0.1 | <0.1 | <0.1 | 0.42 |
| RRT 1.52 | <0.1 | <0.1 | <0.1 | 0.73 |
| RRT 1.65 | <0.1 | 0.12 | 0.25 | 0.62 |
| RRT 1.69 | <0.1 | 0.22 | <0.1 | 0.37 |
| **TOTAL** | **1.35** | **7.84** | **17.83** | **21.85** |

| | | | | |
|---|---|---|---|---|
| means of two injections are reported if >0.1 % | | | | |

**Table 15 - impurity profile of #7 (0.1 M L-aspartic acid, lysine)**

| | **Relative peak area of impurity [%]** | | | |
|---|---|---|---|---|
| **Impurities at 285 nm** | **T0** | **T1m** | **T3m** | **T6m** |
| RRT 0.12 | <0.1 | <0.1 | 0.99 | 1.16 |
| RRT 0.17 | <0.1 | <0.1 | 0.15 | 0.17 |
| RRT 0.43 | <0.1 | <0.1 | <0.1 | 0.11 |
| RRT 0.70 | <0.1 | <0.1 | 0.11 | 0.11 |
| Et-701 | <0.1 | 0.64 | 0.95 | 1.44 |
| RRT 0.90 | <0.1 | 0.15 | 0.18 | 0.29 |
| RRT 0.96 | <0.1 | 0.10 | 0.13 | 0.14 |
| Et-745 | 0.27 | 0.78 | 0.93 | 1.15 |
| Et-759B | 0.34 | 0.63 | 0.68 | 0.58 |
| RRT 1.24 | <0.1 | <0.1 | 0.31 | 0.19 |
| Et-759A | <0.1 | 0.39 | 0.48 | 0.68 |
| RRT 1.59 | <0.1 | <0.1 | <0.1 | 0.12 |
| RRT 1.84 | <0.1 | <0.1 | <0.1 | 0.25 |
| **TOTAL** | **0.61** | **2.69** | **4.91** | **6.39** |

| **Impurities at 255 nm** | **T0** | **T1m** | **T3m** | **T6m** |
|---|---|---|---|---|
| RRT 0.27 | 0.26 | 1.24 | 3.73 | 4.02 |
| RRT 0.31 | 0.30 | 0.43 | 1.63 | 1.08 |
| RRT 0.43 | <0.1 | <0.1 | <0.1 | 0.12 |
| RRT 0.76 | 0.24 | 1.06 | 3.32 | 3.22 |
| RRT 1.31 | <0.1 | <0.1 | <0.1 | 0.19 |
| RRT 1.38 | <0.1 | 0.28 | 0.88 | 0.56 |
| RRT 1.46 | 0.16 | 0.23 | <0.1 | 0.13 |
| RRT 1.52 | <0.1 | <0.1 | <0.1 | 0.30 |
| RRT 1.65 | <0.1 | 0.14 | <0.1 | 0.23 |
| RRT 1.69 | <0.1 | 0.28 | 0.81 | 1.52 |
| **TOTAL** | **0.96** | **3.66** | **10.37** | **11.37** |

| | | | | |
|---|---|---|---|---|
| means of two injections are reported if >0.1 % | | | | |

**Table 16 - impurity profile of #8 (0.1 M L-citrulline, 10 mM citric acid, NaOH)**

| | **Relative peak area of impurity [%]** | | | |
|---|---|---|---|---|
| **Impurities at 285 nm** | **T0** | **T1m** | **T3m** | **T6m** |
| RRT 0.12 | <0.1 | <0.1 | 0.45 | 0.52 |
| Et-701 | <0.1 | 0.10 | 0.16 | 0.20 |
| Et-759B | 0.17 | 0.21 | 0.42 | 0.23 |
| RRT 1.24 | <0.1 | <0.1 | 0.30 | 0.20 |
| RRT 1.84 | <0.1 | <0.1 | <0.1 | 0.25 |
| **TOTAL** | **0.17** | **0.31** | **1.33** | **1.40** |

| **Impurities at 255 nm** | **T0** | **T1m** | **T3m** | **T6m** |
|---|---|---|---|---|
| RRT 0.27 | 0.32 | 0.93 | 1.90 | 1.97 |
| RRT 0.31 | 0.66 | 0.63 | 1.63 | 1.04 |
| RRT 0.76 | 0.28 | 0.75 | 1.42 | 1.38 |
| RRT 1.38 | <0.1 | 0.12 | 0.80 | 0.31 |
| RRT 1.46 | <0.1 | 0.29 | <0.1 | 0.24 |
| RRT 1.65 | <0.1 | 0.11 | 0.11 | 0.27 |
| RRT 1.69 | <0.1 | <0.1 | <0.1 | 0.43 |
| **TOTAL** | **1.26** | **2.83** | **5.86** | **5.64** |

| | | | | |
|---|---|---|---|---|
| means of two injections are reported if >0.1 % | | | | |

**Table 17 - impurity profile of #9 (0.1 M Arg, 10 mM monothioglycerol, 10 mM citric acid, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.12 | <0.1 | <0.1 | <0.1 | 0.10 |
| Et-701 | <0.1 | 0.19 | 0.25 | 0.44 |
| RRT 0.85 | 0.25 | 0.26 | 0.28 | 0.29 |
| Et-759A | <0.1 | <0.1 | <0.1 | 0.12 |
| RRT 1.84 | <0.1 | <0.1 | <0.1 | 0.27 |
| **TOTAL** | **0.25** | **0.45** | **0.53** | **1.22** |

| **Impurities at 255 nm** | **T0** | **T1m** | **T3m** | **T6m** |
|---|---|---|---|---|
| RRT 0.27 | <0.1 | <0.1 | 0.15 | 0.10 |
| RRT 0.31 | 0.13 | 0.13 | 0.24 | 0.16 |
| RRT 1.52 | <0.1 | <0.1 | <0.1 | 0.23 |
| RRT 1.65 | <0.1 | <0.1 | <0.1 | 0.15 |
| RRT 1.69 | <0.1 | <0.1 | <0.1 | 0.15 |
| **TOTAL** | **0.13** | **0.13** | **0.39** | **0.79** |

| | | | | |
|---|---|---|---|---|
| means of two injections are reported if >0.1 % | | | | |

**Table 18 - Impurity profile of #10 (0.1 M Arg, 0.2 % (w/V) L-methionine, 10 mM citric acid, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.12 | <0.1 | <0.1 | 0.53 | 0.59 |
| Et-701 | <0.1 | 0.12 | 0.24 | 0.26 |
| Et-759B | <0.1 | <0.1 | 0.27 | 0.12 |
| RRT 1.24 | <0.1 | <0.1 | 0.25 | 0.18 |
| RRT 1.84 | <0.1 | <0.1 | <0.1 | 0.26 |
| **TOTAL** | **0.00** | **0.12** | **1.29** | **1.41** |

| **Impurities at 255 nm** | **T0** | **T1m** | **T3m** | **T6m** |
|---|---|---|---|---|
| RRT 0.27 | 0.31 | 0.53 | 1.40 | 1.31 |
| RRT 0.31 | 0.32 | 0.20 | 0.90 | 0.56 |
| RRT 0.76 | 0.22 | 0.34 | 1.13 | 0.99 |
| RRT 1.38 | <0.1 | 0.25 | 1.05 | 0.59 |
| RRT 1.52 | <0.1 | <0.1 | <0.1 | 0.20 |
| RRT 1.65 | <0.1 | 0.30 | 0.21 | 0.51 |
| **TOTAL** | **0.85** | **1.62** | **4.69** | **4.16** |

| | | | | |
|---|---|---|---|---|
| means of two injections are reported if >0.1 % | | | | |

**Table 19 - impurity profile of #11 (0.1 M Arg, 0.2 % (w/V) Tween 20, 10 mM citric acid, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.12 | <0.1 | <0.1 | 0.44 | 0.43 |
| Et-701 | <0.1 | 0.13 | 0.26 | 0.31 |
| RRT 0.90 | <0.1 | <0.1 | <0.1 | 0.12 |
| Et-745 | <0.1 | 0.18 | 0.52 | 1.00 |
| RRT 1.20 | <0.1 | <0.1 | <0.1 | 0.10 |
| Et-759B | <0.1 | <0.1 | 0.18 | <0.1 |
| RRT 1.24 | <0.1 | <0.1 | 0.26 | 0.17 |
| RRT 1.31 | <0.1 | <0.1 | <0.1 | 0.25 |
| RRT 1.35 | <0.1 | <0.1 | <0.1 | 0.14 |
| Et-759A | <0.1 | 0.15 | 0.43 | 0.88 |
| RRT 1.51 | <0.1 | <0.1 | <0.1 | 0.10 |
| RRT 1.84 | <0.1 | <0.1 | <0.1 | 0.26 |
| **TOTAL** | **0.00** | **0.46** | **2.09** | **3.76** |

| **Impurities at 255 nm** | **T0** | **T1m** | **T3m** | **T6m** |
|---|---|---|---|---|
| RRT 0.27 | 0.20 | 0.71 | 1.92 | 1.94 |
| RRT 0.31 | 0.50 | 0.59 | 1.21 | 0.93 |
| RRT 0.43 | <0.1 | <0.1 | 0.11 | 0.19 |
| RRT 0.76 | 0.16 | 0.58 | 1.51 | 1.46 |
| RRT 1.38 | <0.1 | 0.16 | 0.44 | 0.19 |
| RRT 1.52 | <0.1 | <0.1 | <0.1 | 0.30 |
| RRT 1.65 | <0.1 | 0.39 | 0.25 | 0.53 |
| RRT 1.69 | <0.1 | <0.1 | 0.22 | 0.24 |
| **TOTAL** | **0.86** | **2.43** | **5.66** | **5.78** |

| | | | | |
|---|---|---|---|---|
| means of two injections are reported if >0.1 % | | | | |

**Table 20 - impurity profile of #12 (0.1 M Arg, 0.1 % (w/V) EDTA, 10 mM citric acid, H₃PO₄)**

| | **Relative peak area of impurity [%]** | | | |
|---|---|---|---|---|
| **Impurities at 285 nm** | **T0** | **T1m** | **T3m** | **T6m** |
| RRT 0.12 | <0.1 | <0.1 | 0.44 | 0.45 |
| Et-701 | <0.1 | 0.23 | 0.29 | 0.37 |
| Et-759B | <0.1 | <0.1 | 0.21 | <0.1 |
| RRT 1.24 | <0.1 | <0.1 | 0.26 | 0.18 |
| RRT 1.84 | <0.1 | <0.1 | <0.1 | 0.28 |
| **TOTAL** | **0.00** | **0.23** | **1.20** | **1.28** |

| **Impurities at 255 nm** | **T0** | **T1m** | **T3m** | **T6m** |
|---|---|---|---|---|
| RRT 0.27 | 0.33 | 0.31 | 1.13 | 0.98 |
| RRT 0.31 | 0.37 | 0.38 | 1.10 | 0.65 |
| RRT 0.76 | 0.24 | 0.24 | 0.90 | 0.72 |
| RRT 1.38 | 0.12 | 0.23 | 1.06 | 0.58 |
| RRT 1.52 | <0.1 | <0.1 | <0.1 | 0.23 |
| RRT 1.65 | <0.1 | 0.22 | 0.16 | 0.38 |
| RRT 1.69 | <0.1 | <0.1 | 0.23 | 0.26 |
| **TOTAL** | **1.06** | **1.38** | **4.58** | **3.80** |

## Claims

1. A composition comprising trabectedin and an amino acid, wherein the weight ratio (w/w) of trabectedin to amino acid is 1:50 to 1:100; and wherein said amino acid is L-arginine.

2. The composition according to claim 1, wherein the weight ratio (w/w) of trabectedin to amino acid is about 1:70, and wherein said amino acid is L-arginine.

3. The composition according to any one of the preceding claims, wherein said composition further comprises a buffering agent, wherein said buffering agent is selected from the group consisting of citric acid, phosphoric acid, acetic acid, basic amino acid and sodium hydroxide, or any mixture thereof, or a mixture of citric acid, phosphoric acid and optionally a basic amino acid.

4. The composition according to any one of the preceding claims, wherein said composition comprises further substances selected from the group comprising a complexing agent such as ethylenediaminetetraacetic acid (EDTA); an antioxidant such as monothioglycerol; a surface-active agent such as polysorbate; and ascorbic acid.

5. A lyophilized formulation comprising trabectedin and an amino acid, wherein the weight ratio (w/w) of trabectedin to amino acid is 1:50 to 1:100, and wherein said amino acid is L-arginine.

6. The lyophilized formulation according to claim 5, wherein the weight ratio (w/w) of trabectedin to amino acid is about 1:70, and wherein said amino acid is L-arginine.

7. The lyophilized formulation according to claim 5 or 6, wherein said composition further comprises a buffering agent, wherein said buffering agent is selected from the group consisting of citric acid, phosphoric acid, acetic acid, basic amino acid and sodium hydroxide, or any mixture thereof, or a mixture of citric acid, phosphoric acid and optionally a basic amino acid.

8. The lyophilized formulation according to any one of claims 5 to 7, wherein said composition comprises further substances selected from the group comprising a complexing agent such as ethylenediaminetetraacetic acid (EDTA); an antioxidant such as monothioglycerol; a surface-active agent such as polysorbate; and ascorbic acid.

9. The lyophilized formulation according to claim 7, wherein said buffering agent is citric acid or phosphoric acid, or a mixture thereof.

10. The lyophilized formulation according to any one of claim 5 to 9, wherein the formulation is provided in a vial, wherein said vial contains 0.1 to 1 mg trabectedin, 10 to 60 mg L-arginine, 0.5 to 8 mg citric acid, 5 to 40 mg phosphoric acid, preferably said vial contains 0.25 mg trabectedin, 17.4 mg L-arginine, 1.9 mg citric acid, and 10 to 15 mg phosphoric acid.

11. A solution for intravenous infusion comprising trabectedin, an amino acid , a buffering agent and water for injection, wherein the weight ratio (w/w) of trabectedin to amino acid is 1:50 to 1:100, and wherein said amino acid is L-arginine.

12. The intravenous infusion solution of claim 11 for use in the treatment of cancer, wherein said cancer is sarcoma, selected from the group of leiomyosarcoma, liposarcoma, osteosarcoma, ovarian cancer, breast cancer, melanoma, colorectal cancer, mesothelioma, renal cancer, endometrial cancer and lung cancer, or any combination thereof.

## Patentansprüche

1. Zusammensetzung, umfassend Trabectedin und eine Aminosäure, wobei das Gewichtsverhältnis (w/w) von Trabectedin zu Aminosäure 1:50 bis 1:100 beträgt; und wobei die Aminosäure L-Arginin ist.

2. Zusammensetzung nach Anspruch 1, wobei das Gewichtsverhältnis (w/w) von Trabectedin zu Aminosäure etwa 1:70 beträgt, und wobei die Aminosäure L-Arginin ist.

3. Zusammensetzung nach einem der vorhergehenden Ansprüche, wobei die Zusammensetzung ferner ein Puffermittel umfasst, wobei das Puffermittel ausgewählt ist aus der Gruppe bestehend aus Zitronensäure, Phosphorsäure, Essigsäure, basischer Aminosäure und Natriumhydroxid oder einer beliebigen Mischung davon, oder einer Mischung aus Zitronensäure, Phosphorsäure und gegebenenfalls einer basischen Aminosäure.

4. Zusammensetzung nach einem der vorhergehenden Ansprüche, wobei die Zusammensetzung weitere Substanzen umfasst, die aus der Gruppe ausgewählt sind, die einen Komplexbildner wie etwa Ethylendiamintetraessigsäure (EDTA); ein Antioxidans wie etwa Monothioglycerin; ein oberflächenaktives Mittel wie etwa Polysorbat; und Ascorbinsäure umfasst.

5. Lyophilisierte Formulierung, umfassend Trabectedin und eine Aminosäure, wobei das Gewichtsverhältnis (w/w) von Trabectedin zu Aminosäure 1:50 bis 1:100 beträgt, und wobei die Aminosäure L-Arginin ist.

6. Lyophilisierte Formulierung nach Anspruch 5, wobei das Gewichtsverhältnis (w/w) von Trabectedin zu Aminosäure etwa 1:70 beträgt, und wobei die Aminosäure L-Arginin ist.

7. Lyophilisierte Formulierung nach Anspruch 5 oder 6, wobei die Zusammensetzung ferner ein Puffermittel umfasst, wobei das Puffermittel ausgewählt ist aus der Gruppe bestehend aus Zitronensäure, Phosphorsäure, Essigsäure, basischer Aminosäure und Natriumhydroxid oder einer beliebigen Mischung davon, oder einer Mischung aus Zitronensäure, Phosphorsäure und gegebenenfalls einer basischen Aminosäure.

8. Lyophilisierte Formulierung nach einem der Ansprüche 5 bis 7, wobei die Zusammensetzung weitere Substanzen umfasst, die aus der Gruppe ausgewählt sind, die einen Komplexbildner wie etwa Ethylendiamintetraessigsäure (EDTA); ein Antioxidans wie etwa Monothioglycerin; ein oberflächenaktives Mittel wie etwa Polysorbat; und Ascorbinsäure umfasst.

9. Lyophilisierte Formulierung nach Anspruch 7, wobei das Puffermittel Zitronensäure oder Phosphorsäure oder eine Mischung davon ist.

10. Lyophilisierte Formulierung nach einem der Ansprüche 5 bis 9, wobei die Formulierung in einer Ampulle bereitgestellt wird, wobei die Ampulle 0,1 bis 1 mg Trabectedin, 10 bis 60 mg L-Arginin, 0,5 bis 8 mg Zitronensäure und 5 bis 40 mg Phosphorsäure enthält, wobei die Ampulle vorzugsweise 0,25 mg Trabectedin, 17,4 mg L-Arginin, 1,9 mg Zitronensäure und 10 bis 15 mg Phosphorsäure enthält.

11. Lösung zur intravenösen Infusion, umfassend Trabectedin, eine Aminosäure, ein Puffermittel und Wasser für Injektionszwecke, wobei das Gewichtsverhältnis (w/w) von Trabectedin zu Aminosäure 1:50 bis 1:100 beträgt, und wobei die Aminosäure L-Arginin ist.

12. Intravenöse Infusionslösung nach Anspruch 11 zur Verwendung bei der Behandlung von Krebs, wobei es sich bei dem Krebs um ein Sarkom handelt, das aus der Gruppe Leiomyosarkom, Liposarkom, Osteosarkom, Eierstockkrebs, Brustkrebs, Melanom, kolorektaler Krebs, Mesotheliom, Nierenkrebs, Endometriumkrebs und Lungenkrebs oder einer beliebigen Kombination davon ausgewählt ist.

## Revendications

1. Composition comprenant de la trabectédine et un acide aminé, dans laquelle le rapport pondéral (w/w) de la trabectédine à l'acide aminé est de 1:50 à 1:100 ; et dans laquelle ledit acide aminé est la L-arginine.

2. Composition selon la revendication 1, dans laquelle le rapport pondéral (w/w) de la trabectédine à l'acide aminé est d'environ 1:70, et dans laquelle ledit acide aminé est la L-arginine.

3. Composition selon l'une quelconque des revendications précédentes, ladite composition comprenant en outre un agent tampon, dans laquelle ledit agent tampon est choisi dans le groupe constitué par l'acide citrique, l'acide phosphorique, l'acide acétique, un acide aminé basique et l'hydroxyde de sodium, ou tout mélange de ceux-ci, ou un mélange d'acide citrique, d'acide phosphorique et éventuellement d'un acide aminé basique.

4. Composition selon l'une quelconque des revendications précédentes, ladite composition comprenant en outre d'autres substances choisies dans le groupe comprenant un agent de complexation tel que l'acide éthylènediaminetétraacétique (EDTA) ; un antioxydant tel que le monothioglycérol ; un agent tensioactif tel que le polysorbate ; et l'acide ascorbique.

5. Formulation lyophilisée comprenant de la trabectédine et un acide aminé, dans laquelle le rapport pondéral (w/w) de la trabectédine à l'acide aminé est de 1:50 à 1:100, et dans laquelle ledit acide aminé est la L-arginine.

6. Formulation lyophilisée selon la revendication 5, dans laquelle le rapport pondéral (w/w) de la trabectédine à l'acide aminé est d'environ 1:70, et dans laquelle ledit acide aminé est la L-arginine.

7. Formulation lyophilisée selon la revendication 5 ou 6, dans laquelle ladite composition comprend en outre un agent tampon, dans laquelle ledit agent tampon est choisi dans le groupe constitué par l'acide citrique, l'acide phosphorique, l'acide acétique, un acide aminé basique et l'hydroxyde de sodium, ou tout mélange de ceux-ci, ou un mélange d'acide citrique, d'acide phosphorique et éventuellement d'un acide aminé basique.

8. Formulation lyophilisée selon l'une quelconque des revendications 5 à 7, dans laquelle ladite composition comprend d'autres substances choisies dans le groupe comprenant un agent de complexation tel que l'acide éthylènediaminetétraacétique (EDTA) ; un antioxydant tel que le monothioglycérol ; un agent tensioactif tel que le polysorbate ; et l'acide ascorbique.

9. Formulation lyophilisée selon la revendication 7, dans laquelle ledit agent tampon est l'acide citrique ou l'acide phosphorique, ou un mélange de ceux-ci.

10. Formulation lyophilisée selon l'une quelconque des revendications 5 à 9, ladite formulation étant fournie dans un flacon, ledit flacon contenant 0,1 à 1 mg de trabectédine, 10 à 60 mg de L-arginine, 0,5 à 8 mg d'acide citrique, 5 à 40 mg d'acide phosphorique, de préférence ledit flacon contenant 0,25 mg de trabectédine, 17,4 mg de L-arginine, 1,9 mg d'acide citrique, et 10 à 15 mg d'acide phosphorique.

11. Solution pour perfusion intraveineuse comprenant de la trabectédine, un acide aminé, un agent tampon et de l'eau pour injection, dans laquelle le rapport pondéral (w/w) de la trabectédine à l'acide aminé est de 1:50 à 1:100 et dans laquelle ledit acide aminé est la L-arginine.

12. Solution de perfusion intraveineuse selon la revendication 11 destinée à être utilisée dans le traitement d'un cancer, ledit cancer étant un sarcome,
choisi dans le groupe constitué par un léiomyosarcome, un liposarcome, un ostéosarcome, le cancer de l'ovaire, le cancer du sein, un mélanome, le cancer colorectal, un mésothéliome, le cancer du rein, le cancer de l'endomètre et le cancer du poumon, ou toute combinaison de ceux-ci.
